(19) Europäisches Patentamt
European Patent Office
Office européen des brevets

(11) **EP 3 470 109 B1**

(12) **EUROPEAN PATENT SPECIFICATION**

(45) Date of publication and mention
of the grant of the patent:
**11.08.2021  Bulletin 2021/32**

(21) Application number: **17813583.6**

(22) Date of filing: **14.06.2017**

(51) Int Cl.:
*A61N 1/32* (2006.01)   *A61N 1/05* (2006.01)
*A61B 1/00* (2006.01)   *A61B 5/053* (2021.01)
*A61B 1/07* (2006.01)   *A61B 1/04* (2006.01)
*A61B 18/00* (2006.01)   *A61B 18/12* (2006.01)
*A61B 18/14* (2006.01)   *A61B 1/018* (2006.01)

(86) International application number:
**PCT/KR2017/006193**

(87) International publication number:
**WO 2017/217760 (21.12.2017 Gazette 2017/51)**

(54) **THERAPEUTIC DEVICE EMPLOYING ENDOSCOPE-INTERWORKING ELECTRODE**

THERAPEUTISCHE VORRICHTUNG MIT ENDOSKOPVERNETZENDER ELEKTRODE

DISPOSITIF THÉRAPEUTIQUE UTILISANT UNE ÉLECTRODE D'INTERFONCTIONNEMENT D'ENDOSCOPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **14.06.2016  KR 20160074045**

(43) Date of publication of application:
**17.04.2019  Bulletin 2019/16**

(73) Proprietor: **Korea University Research and Business Foundation**
**Seoul 02841 (KR)**

(72) Inventors:
• **KIM, Seong-Nam**
**Seoul 06509 (KR)**
• **CHUN, Hoon-Jai**
**Seoul 02822 (KR)**
• **CHOI, Hyuk Soon**
**Seoul 06200 (KR)**
• **KIM, Seung Han**
**Seoul 02843 (KR)**
• **KIM, Eun-sun**
**Seoul 05649 (KR)**
• **KEUM, Bora**
**Seoul 06288 (KR)**
• **LEE, Jae Min**
**Seoul 06276 (KR)**

(74) Representative: **Zacco Sweden AB**
**P.O. Box 5581**
**114 85 Stockholm (SE)**

(56) References cited:
KR-A- 20150 115 223    KR-B1- 101 198 690
US-A1- 2007 232 871    US-A1- 2007 232 871
US-A1- 2011 160 514    US-A1- 2014 081 255
US-A1- 2014 303 452    US-B1- 6 235 023
US-B1- 6 235 023       US-B1- 7 422 585
US-B2- 7 025 765

• AKAHORI H ET AL: "Tube shape piezoelectric 2D microscanner for minimally invasive laser treatment", IEEE INTERNATIONAL CONFERENCE ON MICRO ELECTRO MECHANICAL SYSTEMS, IEEE, US, 30 January 2005 (2005-01-30), pages 76-79, XP010811663, DOI: 10.1109/MEMSYS.2005.1453871 ISBN: 978-0-7803-8732-4
• ESASHI M ET AL: "Biomedical Microsystems for Minimally Invasive Diagnosis and Treatment", PROCEEDINGS OF THE IEEE, IEEE. NEW YORK, US, vol. 92, no. 1, 1 January 2004 (2004-01-01), pages 98-114, XP011105292, ISSN: 0018-9219, DOI: 10.1109/JPROC.2003.820545

**Description**

TECHNICAL FIELD

The present invention relates to a therapeutic device for electrically destroying a target cell in a human body in cooperation with an endoscope.

BACKGROUND ART

Generally, electroporation denotes a therapeutic technique used for injecting a target substance such as intracellular DNA or the like. Specifically, the electroporation aims to form a perforation on a cell membrane through voltage application and inject the target substance through the perforation.

Since the cell membrane is made of lipids, when a high voltage is applied thereto, lipids move to one side, which may result in formation of a perforation on a cell membrane. The perforation can be filled depending on the magnitude of the applied voltage. The case in which the perforation formed through the voltage application is filled as time elapses is referred to as "reversible electroporation."

On the contrary, the case in which the perforation formed through the voltage application is not filled even after time elapses is referred to as "irreversible electroporation." The irreversible electroporation is characterized in that a target cell is necrotized by forming in the target cell a perforation that is not filled even after time elapses.

Potassium ions ($K^+$), which are necessary for controlling intracellular metabolism, exist in cells. If a perforation is formed on a cell membrane, the potassium ions flow out of the cell through the perforation. The cell having an abnormal potassium concentration due to the flow out of the potassium ions receives a signal leading to apoptosis from a receptor on a cell membrane. The apoptosis caused by such irreversible electroporation can be applied to abnormal cells such as cancer cells or malignant tumors.

A conventional electroporation was mostly the reversible electroporation for transmitting a desired substance to a target cell, rather than the irreversible electroporation for necrotizing a target cell. The irreversible electroporation was hardly performed in a human body. This is because, in the irreversible electroporation for necrotizing a target cell in a human body, it is important to accurately set a target cell, but it is considerably difficult to accurately recognize a target cell.

SUMMARY OF THE INVENTION

[Technical Problem]

The present invention provides a therapeutic device capable of performing irreversible electroporation in a human body.

[Technical Solution]

The invention is defined in claims 1 and 4. Further aspects and preferred embodiments are defined in the appended claims. Aspects, embodiments and examples of the present disclosure which do not fall under the scope of the appended claims do not form part of the invention and are merely provided for illustrative purposes.

[Advantageous Effect]

In accordance with the present invention, the electrode capable of performing irreversible electroporation can accurately recognize a target cell in a human body in cooperation with an endoscope.

The irreversible electroporation in accordance with the present invention is advantageous in that side effects of the treatment are reduced because only the target cell is necrotized without affecting tissues.

The therapeutic device in accordance with the present invention can accurately recognize the degree of necrosis of the target cell by using a pair of optical fibers provided in the electrode.

The therapeutic device in accordance with the present invention has therein the configuration capable of recognizing the degree of necrosis of the target cell. Therefore, there is no need to provide an additional inspection device for detecting the degree of necrosis of the target cell. Accordingly, it is not required to remove the therapeutic device and then, insert an additional inspection device in order to monitor the status of the treatment during the treatment. As a result, the treatment efficiency is improved.

BRIEF DESCRIPTION OF THE DRAWINGS

Fig. 1 schematically shows an enlarged view of an end of a therapeutic device in accordance with an embodiment of the present invention.

Fig. 2 schematically shows an enlarged view of an end of a therapeutic device in accordance with another embodiment of the present invention.

Fig. 3 schematically shows an electrode of the therapeutic device in accordance with another embodiment of the present invention.

Fig. 4 shows optical fibers provided in the electrode in accordance with an embodiment of the present invention.

Fig. 5 shows specific examples of electrodes shown in Fig. 2.

Fig. 6 shows other specific examples of the electrodes shown in Fig. 2.

DETAILED DESCRIPTION

The advantages and features of embodiments of accomplishing these will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims. Like reference numerals will designate like parts throughout the specification.

**Therapeutic device according to first embodiment**

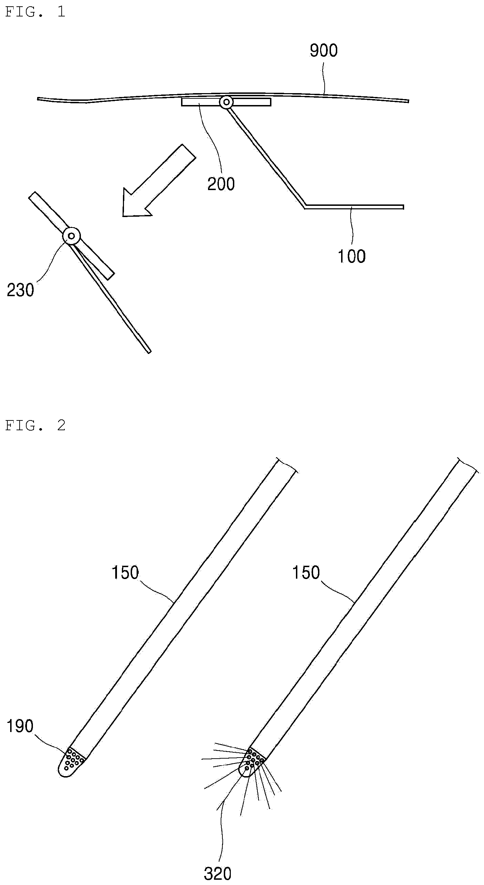

Fig. 1 schematically shows an enlarged view of an end of a therapeutic device in accordance with an embodiment of the present invention. Fig. 2 schematically shows an enlarged view of an end of a therapeutic device in accordance with another embodiment of the present invention.

Referring to Fig. 1, a therapeutic device 10 according to a first embodiment of the present invention includes an endoscope channel 100, and a first electrode 200 that can be inserted into a human body through the endoscope channel 100. The first electrode 200 destroys a target cell 900 in the human body through voltage application. The first electrode 200 includes a first optical fiber 210 and a second optical fiber 220 for detecting the degree of destruction of the target cell through impedance measurement.

Hereinafter, the components of the therapeutic device 10 according to the first embodiment will be described in detail.

Endoscope channel 100

The endoscope channel 100 is inserted into a human body so that an operator can see an inside of a patient's body with naked eyes. The endoscope channel 100 includes a body that can be gripped by a user, an image acquisition unit (not shown) for capturing an image of the inside of the patient's body, a housing (not shown) into which the first electrode 200 can be inserted, and an image output unit for visually outputting the captured image of the inside of the patient's body to the user.

The body of the endoscope channel 100 is formed in a shape that can be easily gripped and manipulated by the user, and has an inner space. The housing into which the first electrode 200 can be inserted, and a wiring that connects the image acquisition unit and the image output unit are provided in the inner space of the body.

When the endoscope channel 100 is inserted into the body of the patient, the body performs a function of protecting the wiring, the first electrode 200 and the like from body fluids of the patient. Therefore, the body needs to be able to withstand acidic body fluids such as gastric acid and the like. Further, the body is brought into direct contact with the inside of the patient's body, and thus should not release chemicals that are harmful to the human body. In order to satisfy the above two conditions, the body can be made of a medical polymer material such as fluorocarbons, polyamide, polyester, polyester elastomer, or the like.

The image acquisition unit is provided at an end of the body which is inserted into the human body. As for the image acquisition unit, it is possible to use various optical devices capable of obtaining an image by receiving light, such as a lens, an optical cable, and the like. The image acquisition unit may further include a light emitting device capable of emitting light to the front side of the body.

The housing is a space for accommodating the first electrode 200. The housing has a diameter that allows the first electrode 200 to pass therethrough. Preferably, the housing has a diameter of 100 to 1000 $\mu$m.

When the housing has a diameter greater than 1000 μm, the body that accommodates the housing becomes excessively large, which makes it difficult to insert the endoscope channel 100 into the patient's body through an esophagus. When the housing has a diameter smaller than 100 μm, the intensity that is enough to protect the electrode may not be ensured. The housing as well as the body need to be able to withstand the acid body fluids such as gastric acid and the like, and should not release chemicals that are harmful to the human body. Therefore, the housing can also be made of a medical polymer material such as fluorocarbons, polyamide, polyester, polyester elastomer, or the like.

The image output unit outputs the image of the inside of the patient's body which has been captured by the image acquisition unit (not shown) so that the user can visually monitor the inside of the patient's body. The image output unit includes a display unit for visual output. The image output unit may be a portable unit that can be carried by a user, or may be a fixed unit such as a computer.

First electrode 200

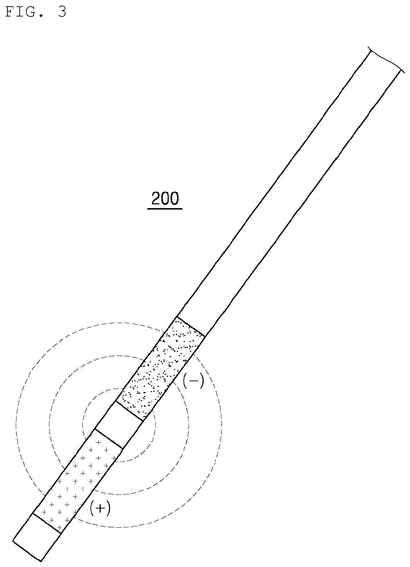

Fig. 3 schematically shows an electrode of the therapeutic device in accordance with another embodiment of the present invention.

Referring to Fig. 3, the first electrode 200 is a device for destroying a target cell through voltage application. Specifically, the first electrode 200 forms a perforation in a cell membrane made of lipids by applying a high voltage to the target cell.

If a sufficiently high voltage is applied to the target cell, the perforation in the cell membrane is not filled even after time elapses. If the perforation permanently exists in the cell membrane, potassium ions ($K^+$) in the cell constantly flow out into the blood. Since the potassium ions control cellular metabolism, cells deficient in the potassium ions cannot perform a function such as cell replication or the like. Such abnormal cells receive a signal leading to apoptosis from a receptor on a cell membrane and result in necrosis.

The above-described series of processes from the voltage application to the target cell is referred to as "irreversible electroporation." The apoptosis caused by the irreversible electroporation can be applied to abnormal cells such as cancer cells or malignant tumors.

In the case of physically excising the abnormal cells such as cancer cells and the like, normal cells or blood vessels in the vicinity thereof may be affected. However, in the case of using the irreversible electroporation, only the target cell is necrotized and, thus, adverse effects on surrounding tissues can be minimized.

The first electrode 200 according to the first embodiment may preferably have a pad shape. The pad-shaped first electrode 200 is brought into close contact with the target cell 900 such as a stomach wall or the like. Since the pad-shaped first electrode 200 is not inserted into the target cell 900, the cell damage caused by the insertion does not occur. For example, in the case of using a therapeutic device on a stomach wall, if the first electrode 200 is inserted into the stomach wall, a scar, i.e., a perforation, from the insertion of the first electrode 200 into the stomach wall is left after the treatment. The perforation can be expanded by gastric acid, which may cause other side effects such as gastric ulcer and the like to the patient.

The first electrode 200 needs to apply a voltage to the stomach wall, and thus should be made of a material having a high electrical conductivity. The first electrode 200 as well as the housing or the body need to be able to withstand acidic body fluids such as gastric acid and the like, and should not release chemicals that are harmful to the human body. Therefore, the first electrode 200 can be preferably made of a metallic material such as 316 stainless steel, Co-Cr alloy, or Ti alloy.

First optical fiber 210 and second optical fiber 220

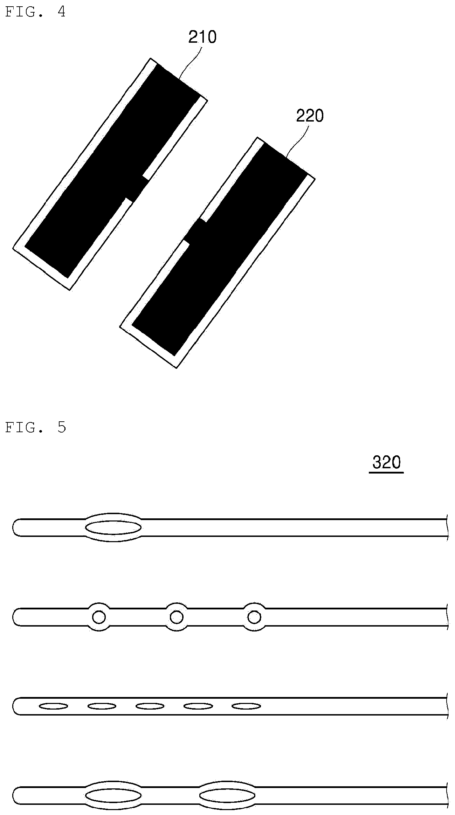

Fig. 4 shows optical fibers provided in the electrode in accordance with an embodiment of the present invention.

Referring to Fig. 4, the first electrode 200 includes a first optical fiber 210 and a second optical fiber 220 for detecting the degree of destruction of the target cell through impedance measurement. In other words, the first optical fiber 210 and the second optical fiber 220 may be provided on one or more surfaces of the first electrode 200.

The first optical fiber 210 and the second optical fiber 220 measure the degree of destruction of the target cell through voltage application by a near-infrared spectroscopy system. The near-infrared rays are emitted from the first optical fiber 210 toward the target cell. The target cell absorbs the near-infrared rays. The degree of absorption of the near-infrared rays may vary depending on the structure of the cell. The near-infrared rays that have passed through the target cell are detected by the second optical fiber 220. Therefore, the user can recognize the degree of cell destruction by comparing the intensity of the near-infrared rays emitted from the first optical fiber 210 and the intensity of the near-infrared rays that have passed through the target cell and detected by the second optical fiber 220. At this time, the impedance is an index for evaluating the degree of absorption of the near-infrared rays.

The near-infrared rays are not easily absorbed by water molecules and blood molecules, and thus can penetrate deep into tissues. Therefore, the near-infrared rays are advantageous in detecting the cell destruction compared to waves in other wavelength ranges. The wavelength range of the waves that can be used as the near-infrared rays is preferably 0.75 to 3 μm). When the wavelength of the waves is less than 0.75 μm, the cell may mutate due to the waves. When the wavelength of the waves is greater than 3 μm, the waves are hardly absorbed by the cell, which makes it difficult to detect the absorption of the waves by the cell depending on the structure of the cell.

The degree of destruction of the target cell is measured by using the near-infrared rays based on the following Beer Lambert law.

[Beer Lambert law]

[Eq. 1]

$$I_{out} = I_{in} 10^{-OD\lambda}$$

$$OD_\lambda = \frac{\log_{10} \frac{I_{out}}{I_{1n}}}{} = \text{attenuation} = A_\lambda + S_\lambda$$

($I_{in}$: incident light, $I_{out}$: emitted light, $OD_\lambda$: optical density, $A_\lambda$: absorbed light, $S_\lambda$: scattered light)

Others

The voltage that is applied to the target cell by the first electrode 200 to perform the irreversible electroporation may be 1 KV or more. If a voltage less than 1 KV is applied to the target cell, the reversible electroporation occurs and a perforation on a target cell membrane may be filled. The target cell being necrotized by forming a permanent perforation on the cell membrane of the target cell cannot be performed by the reversible electroporation.

The first electrode 200 can have a fixed axis 230 connected to the endoscope channel 100 and rotate about the fixed axis 230. For example, when the first electrode 200 is located in the housing 130 of the endoscope channel 100, the first electrode 200 may be oriented in parallel to the housing 130. Accordingly, the diameter of the housing 130 which is required for the first electrode 200 to pass therethrough can be reduced. When the first electrode 200 reaches the target cell, the first electrode 200 can rotate along the outer contour of the target cell. Since the target cell in the human body is mostly curved, the electrode that rotates along the outer curve of the target cell can be brought into closer contact with the curved target cell. Accordingly, the voltage application efficiency is improved.

Referring to Fig. 4, the first optical fiber 210 and the second optical fiber 220 may have optical fiber holes to improve the accuracy of impedance measurement. The optical fiber holes allow the near-infrared rays emitted from the first optical fiber 210 to be directly transmitted to the second optical fiber 220. Accordingly, the errors that may occur in the measurement of the degree of destruction of the target cell due to indirect transmission of the near-infrared rays are decreased.

**Therapeutic device according to second embodiment**

Fig. 5 shows specific examples of the electrode shown in Fig. 2.

Referring to Figs. 2 and 5, a therapeutic device according to a second embodiment includes an endoscope channel, and a catheter that can be inserted into a human body through the endoscope channel. The catheter includes a perforated portion formed on an end thereof, which is inserted into the human body, and a plurality of electrodes that can protrude from the end of the catheter through the perforated portion to penetrate a target cell.

The configuration of the endoscope channel 100 of the device according to the second embodiment of the present invention is substantially the same as that of the endoscope channel 100 of the device according to the first embodiment. Therefore, hereinafter, a catheter 300 according to the second embodiment and a second electrode 320 different from the first electrode will be described in detail.

Catheter 300

The therapeutic device according to the second embodiment of the present invention includes the catheter 300. The catheter 300 includes a perforated portion 310 formed on an end thereof, which is inserted into the human body, and a plurality of electrodes 320 that can protrude from the end of the catheter through the perforated portion 310 to penetrate a target cell.

The catheter 300 needs to be able to withstand acidic body fluids such as gastric acid and the like, and should not release chemicals that are harmful to the human body. Accordingly, the catheter 300 can be made of a medical polymer material such as fluorocarbons, polyamide, polyester, polyester elastomer, or the like.

The perforated portion 310 is provided on an end of the catheter 300. The perforated portion 310 serves as a passage where the second electrodes 320 protrude from the catheter 300. The number of the perforated portions 310 is not limited and can be adjusted, if necessary, by those skilled in the art.

The second electrode 320 according to the second embodiment of the present invention is inserted into the target cell and applies a voltage to the target cell. Therefore, the second electrode 320 can be formed in a shape suitable for insertion, preferably in a probe shape. The second electrode 320 needs to be made of a material having a high electrical conductivity in order to apply a voltage to a stomach wall. Further, the second electrode 320 as well as the catheter 300 need to be able to withstand acidic body fluids such as gastric acid and the like, and should not release chemicals that are harmful to the human body. Therefore, the second electrode 320 can be preferably made of a metallic material such as 316 stainless steel, Co-Cr alloy, or Ti alloy.

The voltage that is applied to the target cell by the second electrode 320 according to the second embodiment of the present invention to perform the irreversible electroporation may be 1 KV or more. If a voltage less than 1 KV is applied to the target cell, the reversible electroporation occurs and a perforation on a target cell membrane may be filled. The target cell being necrotized by forming a permanent perforation on the cell membrane of the target cell cannot be performed by the reversible electroporation.

In accordance with the second embodiment of the present invention, there may be provided a plurality of second electrodes 320. Preferably, the number of the second electrodes 320 may be the same as the number of perforated portions 310.

Each of the second electrodes 320 has both of an anode and a cathode. When a current flows through the second electrode 320 in a state where the second electrode 320 is inserted into the target cell, an electric field is generated between the anode and the cathode in the second electrode 320. A voltage is applied to the target cell due to the electric field thus generated. Since each of the second electrodes 320 of the present invention has both of the anode and the cathode, even if some of the second electrodes 320 are damaged, the treatment can be performed by other second electrodes 320.

The second electrode 320 according to the second embodiment of the present invention may include a first optical fiber and a second optical fiber for detecting the degree of destruction of the target cell through impedance measurement. The description on the first optical fiber and the second optical fiber are the same as that on the first optical fiber 210 and the second optical fiber 220 according to the first embodiment.

Referring to Fig. 5, one or more protrusions having a curved surface may be formed at the second electrode 320. One or more protrusions have a circular or an elliptical surface. In addition, one or more holes penetrating through the second electrode in one or more directions are formed at the second electrode. One or more holes may be formed to correspond to the positions of one or more protrusions. One or more holes may have a circular or an elliptical shape.

Even when the endoscope channel 100 is inserted into a patient's body, especially into a tissue, a target cell, a stomach or the like, the tissue, the target cell, the stomach, or the like keeps working. Thus, the insertion of the second electrode 320 into the tissue, the target cell, the stomach or the like is disturbed, or the second electrode 320 inserted into the tissue, the target cell, the stomach or the like is released therefrom.

However, a surface adhesive strength of the second electrode 320 can be improved by increasing a surface contact area of the second electrode 320 by forming one or more protrusions on the surface of the second electrode 320 or by forming one or more holes at the second electrode 320. If the second electrode 320 is inserted into the tissue, the target cell, the stomach or the like in a state where the surface contact area of the second electrode 320 is increased, an insertion coupling force increases and the efficiency of the treatment can be improved.

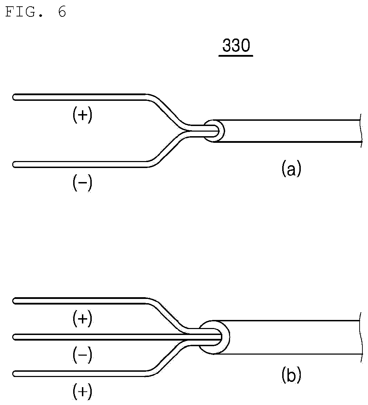

Fig. 6 shows other specific examples of the electrode shown in Fig. 2.

Referring to Figs. 2 and 6, there are provided a plurality of third electrodes 330 of the present invention. Preferably, the number of the third electrodes 330 may be the same as the number of perforated portions 310.

Each of the third electrodes 330 is separated into one or more anodes (+) and one or more cathodes (-) separately. In other words, each of the third electrodes 330 may be configured such that one or more anodes and one or more cathodes are arranged in parallel to each other. When a plurality of anodes (+) and a plurality of cathodes (-) are arranged in parallel to each other, the voltage application efficiency can be improved.

When the third electrode 330 in which a plurality of anodes (+) and a plurality of cathodes (-) are arranged in parallel to each other is inserted into a tissue, a target cells, a stomach or the like and, then, a current flows through the third electrode 330, an electric field is generated between the anodes (+) and the cathodes (-). A voltage is applied to the target cell due to the electric field thus generated. In the case of inserting the third electrode 330 into a tissue, a target cell, a stomach or the like in a state where the anodes (+) and the cathodes (-) are arranged in parallel to each other, an insertion coupling area is increased and, thus, the treatment efficiency can be improved.

One or more protrusions having a curved surface may be formed at the anodes (+) and the cathodes (-) arranged in parallel to each other. One or more protrusions have a circular or an elliptical surface. In addition, one or more holes penetrating through the anodes (+) and the cathodes (-) in one or more directions are formed at the anodes (+) and the cathodes (-). One or more holes may be formed to correspond to the positions of one or more protrusions. One or more holes may have a circular shape or an elliptical shape.

When the surface contact areas of the anodes (+) and the cathodes (-) are increased by forming one or more protrusion or one or more holes on the surfaces of the anodes (+) and the cathodes (-), the surface adhesive strength of the anodes (+) and the cathodes (-) can be improved. If the third electrode 330 is inserted into a tissue, a target cell, a stomach or the like in a state where the surface contact areas of the anodes (+) and the cathodes (-) are increased, the insertion coupling force increases and the efficiency of the treatment can be improved.

While the present invention has been shown and described with respect to the embodiments, it will be understood by those skilled in the art that various changes and modifications may be made without departing from the scope of the present invention as defined in the following claims.

**Claims**

1. A therapeutic device comprising:

    an endoscope channel (100); and
    an electrode (200) inserted into a human body through the endoscope channel,
    wherein the electrode (200) destroys a target cell in the human body through voltage application, and
    the electrode includes a first optical fiber (210) and a second optical fiber (220) for detecting the degree of destruction of the target cell through impedance measurement, wherein the electrode (200) has optical fiber holes to improve accuracy of the impedance measurement between the first optical fiber (210) and the second optical fiber (220).

2. The therapeutic device of claim 1, wherein the electrode (200) applies a voltage of 1 KV or above.

3. The therapeutic device of claim 1, wherein the electrode (200)has a fixed axis connected to the endoscope channel (100) and rotates about the fixed axis.

4. A therapeutic device comprising:

    an endoscope channel (100); and
    a catheter (300) inserted into a human body through the endoscope channel,
    wherein the catheter (300) includes a perforated portion provided on an end thereof, which is inserted into the human body, and a plurality of electrodes that protrudes from the end of the catheter through the perforated portion to penetrate a target cell,
    wherein the electrode includes a first optical fiber and a second optical fiber for detecting the degree of destruction of the target cell through impedance measurement, wherein the electrode has optical fiber holes to improve accuracy of the impedance measurement between the first optical fiber and the second optical fiber.

5. The therapeutic device of claim 4, wherein the electrode destroys the target cell by applying a voltage of 1 KV or above to the target cell.

6. The therapeutic device of claim 4, each of the plurality of electrodes has both of an anode and a cathode.

7. The therapeutic device of claim 4, wherein the plurality of electrodes includes:

    one or more curved protrusions; and
    one or more through-holes.

8. The therapeutic device of claim 4, wherein the plurality of electrodes is separated into an anode and a cathode that are arranged in parallel to each other.

**Patentansprüche**

1.  Therapeutische Vorrichtung umfassend:

    einen Endoskopkanal (100); und
    eine durch den Endoskopkanal in einen menschlichen Körper eingeführte Elektrode (200),
    wobei die Elektrode (200) eine Zielzelle in dem menschlichen Körper durch Anlegen einer Spannung zerstört, und
    die Elektrode eine erste optische Faser (210) und eine zweite optische Faser (220) zum Erfassen des Zerstörungsgrades der Zielzelle durch Impedanzmessung beinhaltet,

    wobei die Elektrode (200) optische Faserlöcher zur Verbesserung der Genauigkeit der Impedanzmessung zwischen der ersten optischen Faser (210) und der zweiten optischen Faser (220) aufweist.

2.  Therapeutische Vorrichtung nach Anspruch 1, wobei die Elektrode (200) eine Spannung von 1 KV oder mehr anlegt.

3.  Therapeutische Vorrichtung nach Anspruch 1, wobei die Elektrode (200) eine mit dem Endoskopkanal (100) verbundene feste Achse aufweist und um die feste Achse dreht.

4.  Therapeutische Vorrichtung umfassend:

    einen Endoskopkanal (100); und
    einen durch den Endoskopkanal in einen menschlichen Körper eingeführten Katheter (300),
    wobei der Katheter (300) einen perforierten Abschnitt, der an einem Ende davon vorgesehen ist, der in den menschlichen Körper eingeführt ist, und eine Vielzahl von Elektroden, die von dem Ende des Katheters durch den perforierten Abschnitt vorstehen, um eine Zielzelle zu durchdringen, beinhaltet,
    wobei die Elektrode eine erste optische Faser und eine zweite optische Faser zum Erfassen des Zerstörungsgrades der Zielzelle durch Impedanzmessung beinhaltet, wobei die Elektrode optische Faserlöcher zur Verbesserung der Genauigkeit der Impedanzmessung zwischen der ersten optischen Faser und der zweiten optischen Faser aufweist.

5.  Therapeutische Vorrichtung nach Anspruch 4, wobei die Elektrode die Zielzelle durch Anlegen einer Spannung von 1 KV oder mehr an die Zielzelle zerstört.

6.  Therapeutische Vorrichtung nach Anspruch 4, wobei jede der Vielzahl von Elektroden sowohl eine Anode als auch eine Kathode aufweist.

7.  Therapeutische Vorrichtung nach Anspruch 4, wobei die Vielzahl von Elektroden beinhaltet:

    einen oder mehrere gekrümmte Vorsprünge; und
    ein oder mehrere Durchgangslöcher.

8.  Therapeutische Vorrichtung nach Anspruch 4, wobei die Vielzahl von Elektroden in eine Anode und eine Kathode getrennt ist, die parallel zueinander angeordnet sind.

**Revendications**

1.  Dispositif thérapeutique comprenant :

    un canal d'endoscope (100) ; et
    une électrode (200) insérée dans un corps humain à travers le canal d'endoscope,
    dans lequel l'électrode (200) détruit une cellule cible dans le corps humain par l'application de tension, et
    l'électrode comprend une première fibre optique (210) et une deuxième fibre optique (220) pour détecter le
    degré de destruction de la cellule cible par la mesure d'impédance,

    dans lequel l'électrode (200) présente des trous de fibre optique pour améliorer la précision de la mesure d'impédance entre la première fibre optique (210) et la deuxième fibre optique (220).

**2.** Dispositif thérapeutique selon la revendication 1, dans lequel l'électrode (200) applique une tension de 1 KV ou plus.

**3.** Dispositif thérapeutique selon la revendication 1, dans lequel l'électrode (200) présente un axe fixe raccordé au canal d'endoscope (100) et tourne autour de l'axe fixe.

**4.** Dispositif thérapeutique comprenant :

un canal d'endoscope (100) ; et
un cathéter (300) inséré dans un corps humain à travers le canal d'endoscope,
dans lequel le cathéter (300) comprend une partie perforée prévue sur son extrémité insérée dans le corps humain, et une pluralité d'électrodes faisant saillie à partir de l'extrémité du cathéter à travers la partie perforée pour pénétrer dans une cellule cible,
dans lequel l'électrode comprend une première fibre optique et une deuxième fibre optique pour détecter le degré de destruction de la cellule cible par la mesure d'impédance, dans lequel l'électrode présente des trous de fibre optique pour améliorer la précision de la mesure d'impédance entre la première fibre optique et la deuxième fibre optique.

**5.** Dispositif thérapeutique selon la revendication 4, dans lequel l'électrode détruit la cellule cible en appliquant une tension de 1 KV ou plus à la cellule cible.

**6.** Dispositif thérapeutique selon la revendication 4, dans lequel chacune de la pluralité d'électrodes présente à la fois une anode et une cathode.

**7.** Dispositif thérapeutique selon la revendication 4, dans lequel la pluralité d'électrodes comprend :

une ou plusieurs saillies incurvées ; et
un ou plusieurs trous traversants.

**8.** Dispositif thérapeutique selon la revendication 4, dans lequel la pluralité d'électrodes est séparée en une anode et une cathode disposées parallèlement l'une à l'autre.

FIG. 1

900

200

230

100

FIG. 2

150

150

190

320

FIG. 3

200

(−)

(+)

FIG. 4

210

220

FIG. 5

320

FIG. 6

330

(+)

(−)

(a)

(+)

(−)

(+)

(b)